(19) **Europäisches Patentamt / European Patent Office / Office européen des brevets**

(11) **EP 2 778 664 A1**

(12) **EUROPEAN PATENT APPLICATION**
published in accordance with Art. 153(4) EPC

(43) Date of publication:
**17.09.2014 Bulletin 2014/38**

(21) Application number: **12848813.7**

(22) Date of filing: **29.10.2012**

(51) Int Cl.:
*G01N 23/201* (2006.01)   *C08K 3/00* (2006.01)
*C08L 21/00* (2006.01)   *G01N 3/00* (2006.01)
*G01N 23/202* (2006.01)   *G01N 33/44* (2006.01)

(86) International application number:
**PCT/JP2012/077888**

(87) International publication number:
**WO 2013/073360 (23.05.2013 Gazette 2013/21)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.11.2011 JP 2011252984**

(71) Applicant: **Sumitomo Rubber Industries, Ltd. Kobe-shi, Hyogo 651-0072 (JP)**

(72) Inventors:
• **KISHIMOTO Hiroyuki**
  **Kobe-shi**
  **Hyogo 651-0072 (JP)**
• **NAITO Masato**
  **Kobe-shi**
  **Hyogo 651-0072 (JP)**

(74) Representative: **Manitz, Finsterwald & Partner GbR**
  **Martin-Greif-Strasse 1**
  **80336 München (DE)**

(54) **METHOD FOR SIMULATING RUBBER MATERIAL**

(57)   Provided is a method that is useful for accurately setting a rubber material model for a simulation from an actual rubber material and obtaining a high-accuracy calculation result. A method for simulating a rubber material containing a filler comprises a measurement step (S1) for measuring scattering data relating to X-rays and/or neutron in the rubber material, a visualization step (S2) for specifying the three-dimensional structure of the filler in the rubber material through a reverse Monte Carlo method from the scattering data, model setting steps (S3 to S6) for setting a rubber material model on the basis of the three-dimensional structure of the filler, and a step for performing a deformation simulation on the basis of the rubber material model, wherein in the measurement step, obtaining the scattering data with a scattering vector (q) within the range of $10^{-4}$nm$^{-1}$ to 10nm$^{-1}$.

**FIG.2**

**Description**

[Technical Field]

The present invention relates to a method for simulating a rubber material, and in particular, relates to a method including accurately setting a rubber material model for a simulation from an actual rubber material so as to be useful for obtaining an accurate computational result.

[Background Art]

From the viewpoint of reinforcement, fillers, such as carbon black, and silica, are blended in a rubber material for tires or the like. It has been roughly found that dispersibility of the fillers in the rubber material considerably affects rubber strength or the like. However, the details thereof are not made so clear. It is therefore important to accurately observe a three-dimensional dispersed state (aggregated structure) of the fillers in the rubber material so as to perform a simulation using a model based on the dispersed state.

With the recent technological developments, it has been proposed to obtain an electron beam transmitted image of the rubber material with a 3D-TEM (transmission electron microscope), and then configure a three-dimensional structure of the rubber material from the image with tomography method so as to set a rubber material model based on the three-dimensional structure.

Unfortunately, all information obtainable with the 3D-TEM are structure information about a local portion among the entirety of rubber, and hence there is the problem that statistical properties are poor for performing the simulation. This leads to deterioration of the accuracy of the simulation.

[Disclosure of the Invention]

[Problems to be Solved by the Invention]

The present invention has been made in view of the foregoing problem, and has an object to provide a method for simulating a rubber material capable of solving the above problem. The method basically includes determining a three-dimensional structure of the rubber material having high statistical properties with reverse Monte Carlo method by using scattering data in a specific scattering vector range obtained using an X-ray and/or neutron of the rubber material, and setting a rubber material model based on the three-dimensional structure.

[Means of Solving the Problems]

The present invention is a method for simulating a rubber material containing fillers. The method includes a measuring step of measuring scattering data of an X-ray and/or neutron of the rubber material, a visualization step of determining a three-dimensional structure of the fillers in the rubber material with reverse Monte Carlo method by using the scattering data, a model setting step of setting a rubber material model based on the three-dimensional structure of the fillers, and a step of performing a deformation simulation based on the rubber material model. The measuring step includes obtaining scattering data in a range in which a scattering vector (q) expressed by equation (1) is greater than $10^{-4}$ nm$^{-1}$ and less than 10 nm$^{-1}$, $q = 4\pi \cdot \sin\theta/\lambda$ ... (1), wherein $\lambda$ is a wavelength of an electromagnetic wave or particle beam, and $\theta$ is a half of a scattering angle.

In the measuring step, a beam size of an X-ray and/or neutron ray entering a sample is preferably equal to or more than 60 $\mu$m and equal to or less than 30 mm.

In the measuring step, incident X-ray intensity to be measured with an X-ray scattering method is equal to or more than $10^{10}$ (photons/s/mrad$^2$/mm$^2$/0.1% bw) and equal to or less than $10^{23}$ (photons/s/mrad$^2$/mm$^2$/0.1% bw).

[Effects of the Invention]

The method for simulating a rubber material according to the present invention includes the measuring step of measuring scattering data of the X-ray and/or neutron of the rubber material, the visualization step of determining a three-dimensional structure of fillers in the rubber material with the reverse Monte Carlo method by using the scattering data, the model setting step of setting a rubber material model based on the three-dimensional structure of the fillers, and the step of performing a deformation simulation based on the rubber material model. The measuring step includes obtaining scattering data in the range in which the scattering vector (q) expressed by the following equation is greater than $10^{-4}$ nm$^{-1}$ and less than 10 nm$^{-1}$, $q = 4\pi \cdot \sin\theta/\lambda$, wherein $\lambda$ is a wavelength of an electromagnetic wave or particle beam, and $\theta$ is a half of a scattering angle.

In general, the fillers (reinforced fillers), such as silica, for use in rubber have a primary particle size of approximately 10 to 100 nm. A primary aggregated body that a plurality of particles of the fillers aggregate generally has a size of approximately 500 nm or less. On the other hand, the scattering vector relates to space dissolution obtained by a computation with the reverse Monte Carlo method. Therefore, in the case of using a large scattering vector relative to the primary particle size of the fillers or the size of the primary aggregated body thereof, this case leads to a computation with unnecessary space dissolution, resulting in poor efficiency. Reversely, in the case of using a small scattering vector, though this case allows observation even with a scanning electron microscope (SEM) and an optical microscope, this case is not practical because much computation costs are required. With the present invention, the scattering vector (q) is limited to the above-mentioned range, thereby ensuring efficient and accurate determination of the shape of the primary aggregated body and the layout of the primary particles in the fillers.

Hence, according to the foregoing steps, it is ensured to accurately determine a three-dimensional structure that the actual rubber material actually has, thereby obtaining a more accurate rubber material model based on the three-dimensional structure. Thus, the present invention ensures an accurate simulation result.

[Brief Description of the Drawings]

[FIG. 1] FIG. 1 is a schematic partially enlarged cross-sectional view of a rubber material according to an embodiment of the present invention.
[FIG. 2] FIG. 2 is a flow chart describing the procedure according to the embodiment.
[FIG. 3] FIG. 3 is a three-dimensional image of the rubber material obtained from a sample with a method according to the embodiment.
[FIG. 4] FIG. 4(a) is a partially enlarged view of a two-dimensional rubber material model; FIG. 4(b) is an enlarged view of a main part thereof.
[FIG. 5] FIG. 5(a) is a partially enlarged view of another two-dimensional rubber material model; FIG. 5(b) is an enlarged view of a main part thereof.
[FIG. 6] FIG. 6 is a schematic enlarged view of a part of a three-dimensional rubber material model.
[FIG. 7] FIG. 7 is an enlarged view of a cubic element describing subdivision of the cubic element.

[Mode for Carrying out the Invention]

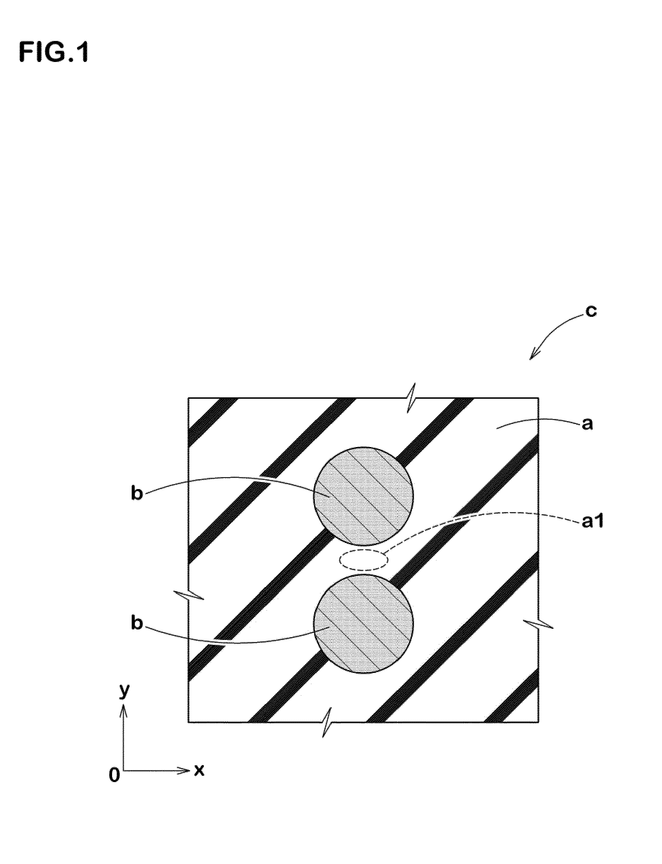

An embodiment of the present invention will be described below with reference to the drawings. In the present embodiment, as shown in FIG. 1, an analysis object is a rubber material (c) with fillers containing rubber ingredients (a) as matrix rubber, and silica as fillers (b). A deformation computation of the rubber material (c) is simulated with a computer (not shown).

Examples of the rubber ingredients (a) include natural rubber (NR), isoprene rubber (IR), butyl rubber (IIR), butadiene rubber (BR), styrene butadiene rubber (SBR), styrene isoprene butadiene rubber (SIBR), ethylene propylene diene rubber (EPDM), chloroprene rubber (CR), and acrylonitrile butadiene rubber (NBR).

Examples of the fillers (b) include, without limitation to silica, carbon black, clay, talc, magnesium carbonate, and magnesium hydroxide. The rubber material (c) may be suitably blended with various materials generally used in rubber industry, such as sulfur, and vulcanization accelerator.

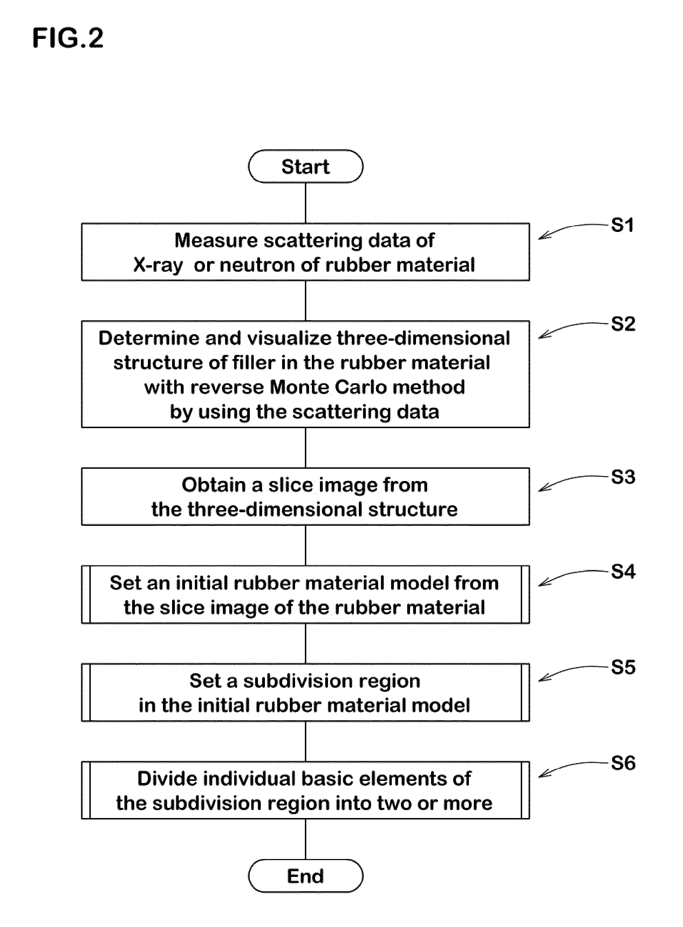

FIG. 2 shows a flow chart for performing a simulation method of the present embodiment. In the present embodiment, firstly, a measuring step of measuring scattering data of an X-ray and/or neutron of the rubber material (c) is performed (step S1).

The measuring step is performed with, for example, small angle scattering method. With the small angle scattering method, the X-ray or neutron is irradiated to the rubber material. An incident X-ray reflects information about an electron density distribution within material (the distribution of the fillers in the present embodiment), and a scattering X-ray (or scattering neutron) occurs around the incident X-ray (or the neutron ray). That is, in the presence of a non-uniform region of particles and density in the rubber material, scattering occurs around the incident X-ray irrespective of whether crystalline or amorphous. The scattering X-ray exposes, for example, a detector so as to form an X-ray latent image corresponding to the scattering data in the interior of the detector. The X-ray latent image is visualized to obtain three-dimensional structure information about the fillers.

The measuring step is performed in a radiation light research facility, such as SPring-8, and PF. In the present embodiment, the measurement was made with the small angle X-ray scattering method using two kinds of beamlines of BL20XU and BL40B2 in the SPring-8. As the detector, an X-ray image intensifier plus a CCD detector (produced by Hamamatsu Photonics K.K.), and a solid-state semiconductor detector PILATUS 100K (produced by DECTRIS Ltd.) were used. The use of these two beamlines ensures obtaining scattering data in the range in which the scattering

vector expressed by the following equation (1) is greater than $10^{-4}$ nm$^{-1}$ and less than 10 nm$^{-1}$. With the present embodiment, two-dimensional small-angle X-ray scatteri ng data in the range of $1.2 \times 10^{-3}$ nm$^{-1}$ < q < 2 nm$^{-1}$ with respect to a scattering vector (q) were obtained.

$$q = 4\pi \cdot \sin\theta/\lambda \ \dots \ (1),$$

wherein $\lambda$ is a wavelength of an electromagnetic wave or particle beam, and $\theta$ is a half of a scattering angle.

The fillers (reinforced fillers) for use in rubber preferably have a primary particle size of approximately 10 to 100 nm. A primary aggregated body that a plurality of particles of the fillers aggregate preferably generally has a size of approximately 500 nm or less. On the other hand, the scattering vector (q) relates to space dissolution obtained from a computation with the reverse Monte Carlo method. Therefore, in the case of using a large scattering vector relative to the primary particle size of the fillers or the si ze of the pri mary aggregated body thereof, this case leads to a computation with unnecessary space dissolution, resulting in poor efficiency. Reversely, in the case of using a small scattering vector, though this case allows observation even with a scanning electron microscope (SEM) and an optical microscope, this case is not practical because much computation costs are required. With the present embodiment, the range of the scattering vector (q) is limited to the above-mentioned range, thereby producing the advantage of ensuring efficient and accurate determination of the shape of the primary aggregated body and the layout of the primary particles. The range of the scatteri ng vector (q) is more preferably $10^{-4}$ nm$^{-1}$ < q < 1 nm$^{-1}$, and still more preferably $10^{-3}$ nm$^{-1}$ < q < 0.7 nm$^{-1}$.

In the measuring step, the beam size of an X-ray and/or neutron ray entering a rubber material (sample) is preferably in the range of 60 $\mu$m or more and 30 mm or less. As structure information obtained from the scattering of the X-ray or neutron ray, average information in the beam size of the X-ray or neutron entered the sample are obtainable, thus ensuring data having a higher statistic than a 3D-TEM.

In the reverse Monte Carlo method (described later), the beam size of 60 $\mu$m or more is preferably irradiated to the sample in order to compute scattering data of the scattering vector (q) of $10^{-4}$ nm$^{-1}$ < q < 10 nm$^{-1}$. When the beam size is less than 60 $\mu$m, the statistic of the scattering data is small relative to a desired structure size, resulting in a risk of failing to accurately determine a space layout of the fillers. Further, in the case of usi ng a synchrotron radi ati on X-ray as an incident X-ray light source, the employment of the beam size of less than 60 $\mu$m causes a speckl e-shaped scatteri ng pattern due to the influence of X-ray space coherence. The speckle-shaped scattering pattern constitutes a noise composition, thus being unsuitable for performing the reverse Monte Carlo method. On the other hand, when the beam size is greater than 30 mm, it is difficult to form an optimum optical system, resulting in a risk of smearing (image blur) of a scattering pattern.

In the measuring step, incident X-ray intensity measured with X-ray scattering method is preferably in the range of $10^{10}$ (photons/s/mrad$^2$/mm$^2$/0.1% bw) or more and $10^{23}$ (photons/s/mrad$^2$/mm$^2$/0.1% bw) or less. Incident X-ray brightness considerably relates to an S/N ratio of the X-ray scattering data. When the incident X-ray brightness is less than $10^{10}$ (photons/s/mrad$^2$/mm$^2$/0.1% bw), there is a tendency that signal intensity is weaker than a statistical error in the X-ray. It is difficult to obtain data of a sufficiently satisfactory S/N ratio even with a longer measuring time. On the other hand, when the incident X-ray brightness is more than $10^{23}$ (photons/s/mrad$^2$/mm$^2$/0.1% bw), there is a risk that the sample is subjected to radiation damage, failing to make the measurement. In view of the foregoing, the incident X-ray intensity is more preferably $10^{21}$ (photons/s/mrad$^2$/mm$^2$/0.1% bw) or less, and still more preferably $10^{20}$ (photons/s/mrad$^2$/mm$^2$/0.1% bw) or less.

Subsequently, with the present embodiment, a visualization step of determining a three-dimensional structure of the fillers in the rubber material with the reverse Monte Carlo method by using the scattering data obtained in the measuring step is performed (step S2).

The reverse Monte Carlo method is the method whose study has been advanced as a technique for determining the atomic and molecular structures of an amorphous material, such as liquid metal. In general , scattering intensity I(q) obtained from the X-ray and/or neutron ray is expressed by the following equation (2).

$$I(q) = S(q) \cdot F(q) \ \dots \ (2)$$

Here, F(q) is a function indicating the shape of a scattering body in a material. In the present embodiment, the primary particles of the fillers in the rubber are denoted by F(q). When F(q) is a shape factor of the primary particles of the fillers, S(q) becomes one related to the space layout of the primary particles. Here, a scattering factor for a sphere is used as the F(q). The scattering factor is expressed by the following equation (3). In the equation (3), R is a radius of a sphere, $\Delta\rho$ is an electron density difference, V is a volume of the sphere, and (q) is a scattering vector.
[Equation 1]

$$F(q) = (\Delta\rho V)^2 \frac{(3\sin(qR) - qR\cos(qR))^2}{(qR)^6} \quad \cdots (3)$$

With the technique of the reverse Monte Carlo method, a plurality of particles are subjected to an initial arrangement in a computer, and a computation is repeated while changing the arrangement of the particles by using random numbers or the like until 1 an $S_{cal}(q)$ computationally obtained from Fourier transformation of the initial arrangement coincides with an actual measurement $S_{exp}(q)$. Actually, a structure is determined by repeating the computation until the computation of $x^2$ shown in equation (4) converges. In the equation (4), $\sigma^2_{std}$ is a standard deviation.
[Equation 2]

$$\chi^2 = \frac{\sum_q (S_{exp}(q) - S_{cal}(q))^2}{\sigma^2_{std}} \quad \cdots (4)$$

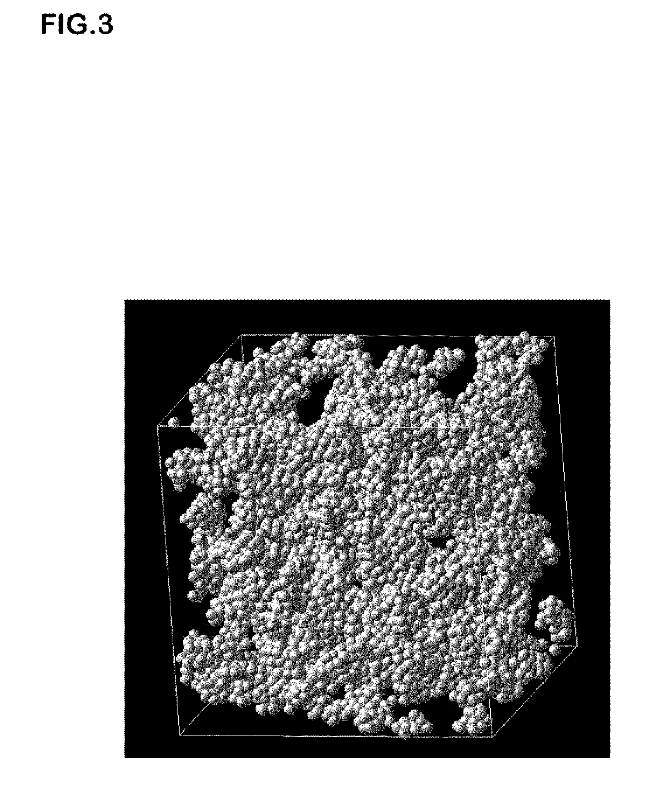

FIG. 3 shows a three-dimensional structure of the fillers in the rubber determined with the reverse Monte Carlo method described above. The three-dimensional structure is stored on the computer as numeric data.

Subsequently, with the present embodiment, the step of obtaining a slice image of the rubber material (c) from the three-dimensional structure is performed (step S3). The slice image can easily be output from the computer by designating a cross-sectional position because the three-dimensional structure related to the fillers in the rubber material (c) has already been obtained.

Subsequently, with the present embodiment, the step of setting an initial rubber material model from the slice image of the rubber material (c) is performed (step S4). This step includes dividing the entire region of the slice image into at least two of the rubber ingredients and the fillers by performing an image processing of the slice image. This type of image processing is already well known. The computer automatically classifies individual regions of the slice image into a rubber part and a filler part by previously setting a threshold value to information, such as lightness and brightness of the image.

Then, after the slice image is classified into the rubber part and the filler part by the image processing, an initial rubber material model is set by dividing the slice image by basic elements of identical shape respectively defined by ordered grids.

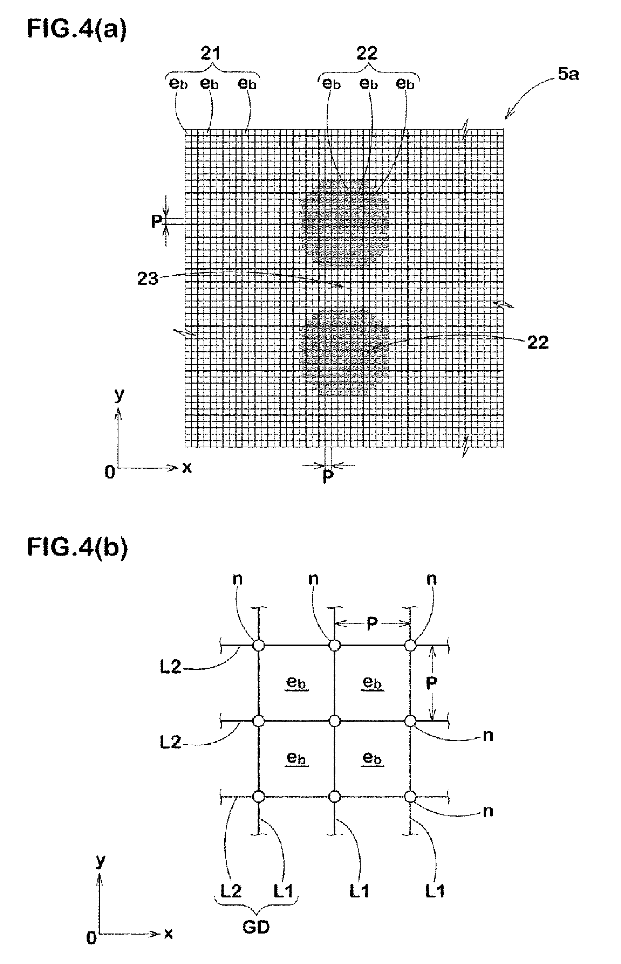

FIG. 4(a) shows a visualized part of an initial rubber material model 5a of the present embodiment. FIG. 4(b) shows a partially enlarged view thereof. The ordered grids are made of grids GD defined by vertical lines L1 and lateral lines L2 arranged at an identical pitch P on the x-axis and y-axis as shown in enlarged dimension in FIG. 4(b). Squares defined by the vertical lines L1 and the lateral lines L2 respectively constitute individual basic elements (eb). More specifically, each of the basic elements (eb) is a square element (rectangular element) whose four corners correspond to nodes (n) arranged at individual intersections of the vertical lines L1 and the lateral lines L2.

The initial rubber material model 5a of the present embodiment includes rubber models 21 resembling the rubber ingredients (a), and filler models 22 resembling the fillers (b).

The filler models 22 are colored and shown in FIG. 4(a) in an easy-to-understand manner. The filler models 22 are set by discretization of the fillers (b) using a finite number of basic elements (eb).

The rubber models 21 are set by discretization of the rubber ingredients (a) of the rubber material (c) using the finite number of basic elements (eb).

In the foregoing element division, for example, using the computer, the ordered grids are set on the slice image after subjected to the image processing, and a computation is performed for each of the basic elements (eb) as to whether the rubber ingredients (a) or the fillers (b) occupies a larger area. Then, a determination whether the individual elements (eb) belong to the rubber model 21 or the filler model 22 is made based on computational results. Thus, an initial analysis model can be created in a short time by using only the basic elements (eb) defined by the ordered grids. The initial analysis model is also set as one that extremely resembles an analysis object owing to the use of the accurately taken slice image of the three-dimensional structure of the rubber material 5.

Information necessary for a numerical analysis owing to a simulation are defined on the basic elements (eb). The numerical analysis denotes, for example, a numerical analysis method, such as finite element method. The information necessary for the analysis include at least the numbers of the nodes (n) constituting the individual basic elements (eb), and coordinate values of the nodes (n). Further, a material property (physical property value) or the like of a portion represented by each of the basic elements (eb) is defined on the basic elements (eb). That is, a material constant according to the physical properties of the fillers and the rubber is defined on each of the basic elements (eb) of the rubber models 21 and the filler models 22. Then, all these pieces of information are input into and stored in the computer.

Subsequently, with the present embodiment, a subdivision region 23 to further divide the basic elements (eb) is set in a part of the initial rubber material model 5a (step S5).

The subdivision region 23 is the portion of the rubber material model 5a that is made of elements smaller than the basic elements (eb). Therefore, the subdivision region 23 ensures a more detailed investigation of deformation behavior thereof, and also ensures high computational accuracy. Hence, the subdivision region 23 is preferably set to the portion that meets these requirements.

In the case of the rubber material (c) blended with the fillers, the rubber part (a1) between the fillers (b) and (b) adjacent to each other is susceptible to large strain and stress as shown in FIG. 1. Therefore, with the present embodiment, the rubber part disposed between the filler models 22 and 22 is set as the subdivision region 23 so as to at least partially include the rubber part (a1).

The range of the subdivision region 23 may be designated by a user with input means, such as a keyboard, and a mouse. Then, predetermined information are added to the elements in a region designated as the subdivision region 23, and the results are input into the computer.

The subdivision region 23 may be determined with a different method. For example, first, a deformation simulation is performed using the initial rubber material model 5a and based on a predetermined deformation condition. From the result of the deformation simulation, a large deformation region including a portion of the initial rubber material model 5a subjected to the maximum stress or strain may be determined, and a region at least partially including the large deformation region may be determined as the subdivision region 23.

Subsequently, with the present embodiment, subdivision for dividing individual basic elements of the subdivision region 5 into two or more is performed (step S6).

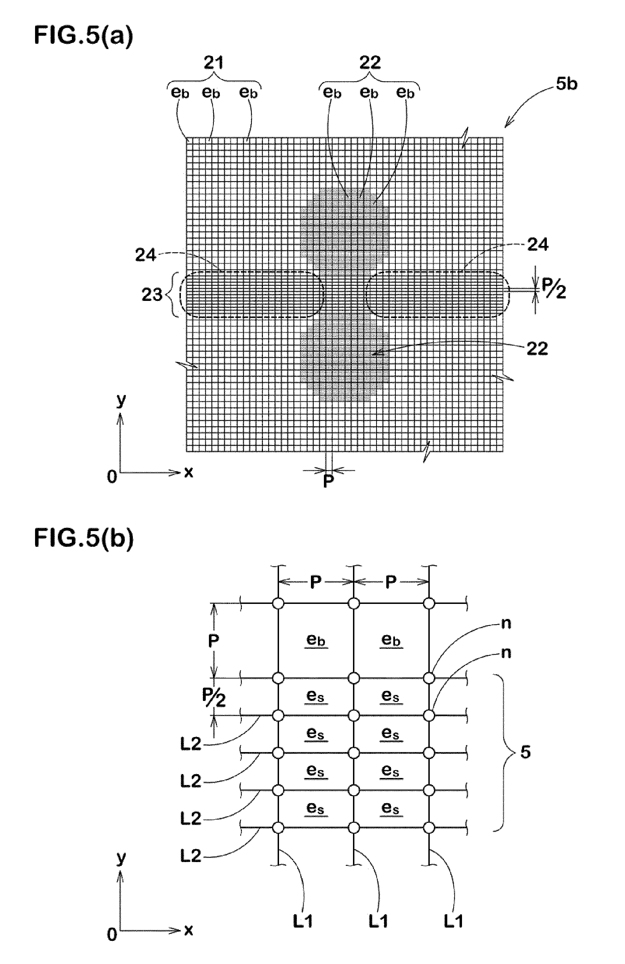

The step S6 for the subdivision is performable by, for example, reducing the pitch P of the vertical lines L1 and/or the lateral lines L2 corresponding to the ordered grids passing through the subdivision region 23 so as to reduce the basic elements (eb). With the present embodiment, only the pitch of the lateral lines L2 defining the ordered grids and passing through the subdivision region 23 is reduced to half of the pitch P that has been determined for the initial rubber material model 5a, as shown in FIG. 5(a) and FIG. 5(b), which is a partially enlarged view thereof. Consequently, the basic elements (eb) of the rubber part disposed between the filler models 22 and 22 are respectively divided into two equal parts in the y-direction. That is, the individual basic elements (eb) of the subdivision region 23 are respectively divided into rectangular small elements (es) having an identical x-dimension and a half y-dimension when compared with the original basic elements (eb).

Accordingly, by performing the deformation simulation using the analysis model 5b after subjected to the step S6 for the subdivision, it is ensured to enhance the computational accuracy of the rubber part between the filler models 22 and 22 (the subdivision region 23), and it is also ensured to investigate in detail of the deformation behavior of the rubber part. The change of the pitch when performing the subdivision is not limited to the reduction by half, but the pitch is settable to different values. The step S3 for the subdivision may be repeated a plurality of times until obtaining a necessary element resolution.

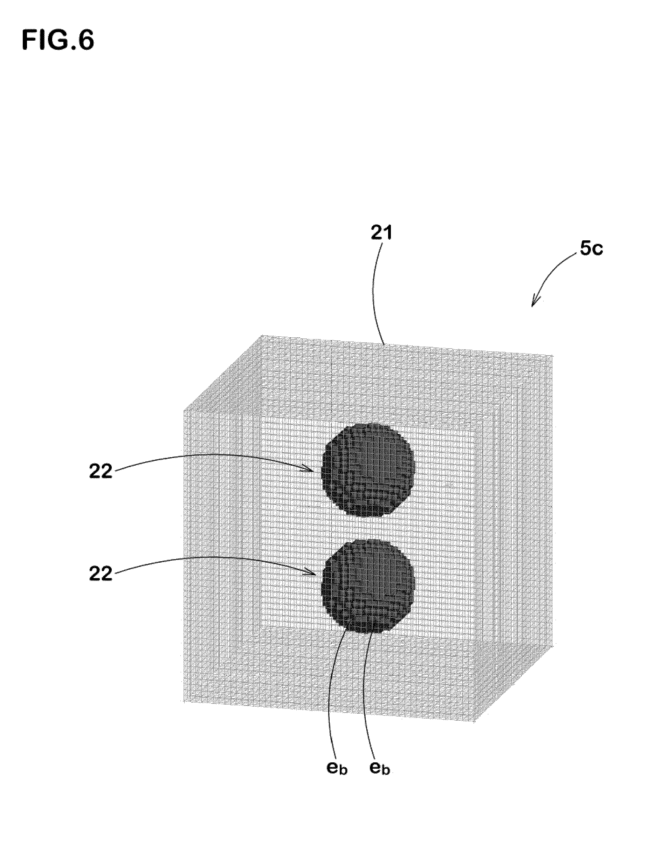

Although the foregoing embodiment has illustrated and described the two-dimensional rubber material model 5a, the present invention is of course applicable to a three-dimensional rubber material model 5c with a similar procedure as shown in FIG. 6. In this case, modeling is performable directly from the three-dimensional structure of the rubber material (c) without using the slice image. In the case of the three-dimensional model 5c, the basic elements (eb) defined by the ordered grids are respectively made of a rectangular parallelepiped element.

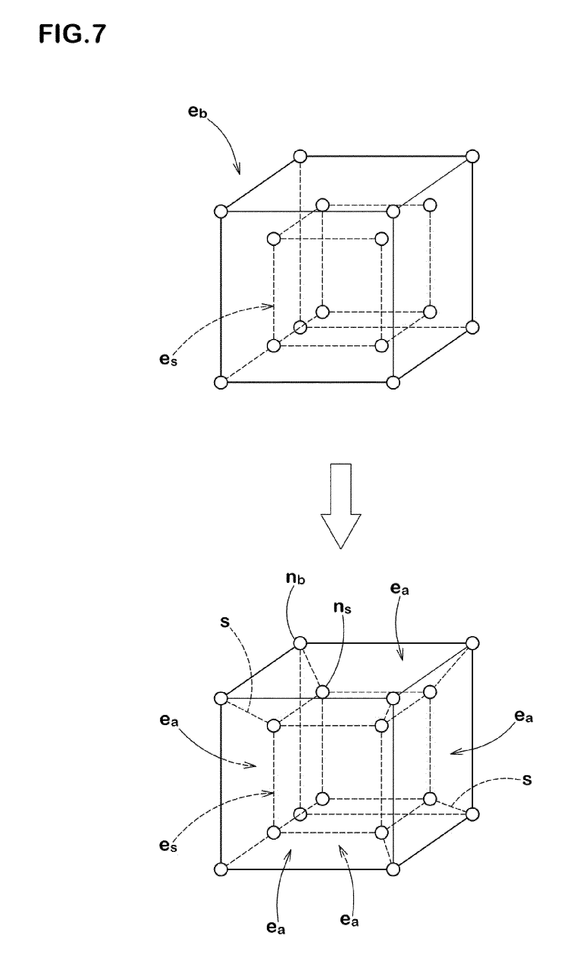

For example, in the step of subdividing the three-dimensional rubber material model 5c, a cubic small element (es) that has a similar shape to the basic element (eb) and is smaller than the basic element (eb) is set in the interior of the basic element (eb) made of a cube so that their respective centers of gravity coincide with each other as shown in the upper diagram in FIG. 7. Then, individual nodes (ns) of the small elements (eb) are respectively coupled to nodes (nb) of the basic element (eb) via a side (s) as shown in the lower diagram in FIG. 7. Consequently, the basic element (eb) can be divided into the single cubic small element (es) and six hexahedral elements (ea) surrounding the small element (es).

While the present invention has been described in detail, the present invention is not limited to the foregoing embodiment, but can be of course modified and carried out in various aspects. For example, a similar measuring step can be performed with the neutron ray instead of the X-ray.

[Examples]

The following test was conducted to confirm the effect of the present invention. However, the present invention is not limited to the following examples.

various kinds of chemicals and devices used in the test are as follows. That is, according to the following blending, materials other than sulfur and vulcanization accelerator were mixed with a Banbury mixer for four minutes under the condition that discharge temperature was 160°C, thereby obtaining a mixture. Then, sulfur and vulcanization accelerator were added to the obtained mixture. This was kneaded with an open role for two minutes under the condition of 100°C, thereby obtaining an unvulcanized rubber composition. The obtained unvulcanized rubber composition was vulcanized for 30 minutes at 175°C, thereby obtaining a vulcanized rubber.

| [Rubber Blending] | (unit: parts by mass) |
| --- | --- |
| SBR | 100 |
| Silica | 50 |
| Silane Coupling Agent | 4 |
| Sulfur | 2 |
| Vulcanization Accelerator A | 1 |

[Chemicals]

SBR: "SBR1502" produced by Sumitomo Chemical Co., Ltd.
Silica: "115Gr" produced by RHODIA JAPAN LTD.
Silane Coupling agent: "Si69" produced by Degussa AG
Sulfur: Powder Sulfur produced by TSURUMI CHEMICAL INDUSTRY CO., LTD.
vulcanization accelerator A: "Nocceler NS" produced by OUCHI SHINKO CHEMICAL INDUSTRIAL CO., LTD.

Then, a 1-mm-thi ck slab sheet was cut out of the vulcanized rubber as a sample. When the sample has a thickness greater than 1 mm, there is a risk that multiple scattering occurs within the rubber material, failing to make an accurate measurement.

Subsequently, a scattering data measurement for the obtained sample was made with the small angle X-ray scattering method according to the specification presented in Table 1 by using beamlines of BL20XU and BL40B2 in the SPring-8. As detectors, the X-ray image intensifier plus the CCD detector (produced by Hamamatsu Photonics K.K.), and the solid-state semiconductor detector "PILATUS 100K" (produced by DECTRIS Ltd.) were used as described above. Scattering data in the range greater than $10^{-4}$ nm$^{-1}$ and less than 1 nm$^{-1}$ were obtained by using the foregoing two beamlines. The three-dimensional structure of silica was determined from the obtained scattering data with the reverse Monte Carlo method. Based on the three-dimensional structure of silica, a rubber material model , whose one side was 700 nm, was set, and a deformation simulation in which the model was subjected to a 100% pull with finite element method was performed. Then, a 100% modulus of the rubber material was computed as an evaluation. For comparison, an actual vulcanized rubber was also subjected to a 10% modulus pull test under the same condition (comparati ve examples). The test resul ts and the like are presented in Table 1. The values of the 10% modulus in Table 1 are ones obtained when the comparative exampl es are taken as 100, i n whi ch value closer to 100 indicates being closer to the actual measurement.

[Table 1]

| | Example 1 (Simulation Result) | Example 2 (Simulation Result) | Example 3 (Simulation Result) | Comparative Example 1 (Simulation Result) | Comparative Example 2 (simulation Result) | Comparative Example 3 (Simulation Result) |
| --- | --- | --- | --- | --- | --- | --- |
| Range of q(nm$^{-1}$) Used for Reverse Monte Carlo Method | $10^{-3}$ to 1 | $10^{-3}$ to 1 | $10^{-3}$ to 1 | 10 to 20 | $7 \times 10^{-5}$ to $10^{-4}$ | - |
| Incident X-ray Intensity (photons/s/mra d$^2$/mm$^2$/0.1% bw) | about $10^{19}$ | about $10^{19}$ | about $10^{9}$ | about $10^{19}$ | about $10^{19}$ | - |
| Beam Size Diameter ($\mu$m) | 500 | 5 | 500 | 500 | 100 | - |

(continued)

| | Example 1 (Simulation Result) | Example 2 (Simulation Result) | Example 3 (Simulation Result) | Comparative Example 1 (Simulation Result) | Comparative Example 2 (simulation Result) | Comparative Example 3 (Simulation Result) |
|---|---|---|---|---|---|---|
| Computational Possibility for X-ray Scattering | Possible | Possible | Possible | Possible | Possible | - |
| Computational Possibility with Reverse Monte Carlo Method | Possible | Possible | Possible | Impossible | Impossible | - |
| Simulation Result [10% Modulus (index)] | 98 | 93 | 94 | Not Performed | Not Performed | 100 |

These examples have high correlation with the actual vulcanized rubber. In contrast, comparative example 1, which employed the scattering vector (q) smaller than the particle size of silica, failed to obtain a convergent solution in the computation with the reverse Monte Carlo method. In comparative example 2, the scattering vector (q) fell within the range smaller than the range of the present invention, and hence the computation scale was too large to perform the computati on wi th the reverse Monte Carlo method.

Description of the Reference Numeral

5a, 5b, 5c    Rubber material models
21            Rubber model
22            Filler model

**Claims**

1.  A method for simulating a rubber material containing a filler, the method comprising:

    a measuring step of measuring scattering data of an X-ray and/or a neutron of the rubber material;
    a visualization step of determining a three-dimensional structure of the filler in the rubber material with reverse Monte Carlo method by using the scattering data;
    a model setting step of setting a rubber material model based on the three-dimensional structure of the filler; and
    a step of performing a deformation simulation based on the rubber material mode,
    wherein the measuring step includes obtai ni ng scatteri ng data in a range in which a scattering vector (q) expressed by equation (1) is greater than $10^{-4}$ nm$^{-1}$ and less than 10 nm$^{-1}$,

    $$q = 4\pi \cdot \sin\theta/\lambda \quad (1),$$

    wherein $\lambda$ is a wavelength of an el ectromagneti c wave or particle beam, and $\theta$ is a half of a scattering angle.

2.  The method for simulating a rubber material according to claim 1,
    wherein in the measuring step, a beam size of the X-ray and/or neutron entering a sample is equal to or more than 60 $\mu$m and equal to or less than 30 mm.

3.  The method for simulating a rubber material according to claim 1 or 2,
    wherein in the measuring step, incident X-ray intensity to be measured with an X-ray scattering method is equal to or more than $10^{10}$ (photons/s/mrad$^2$/mm$^2$/0.1% bw) and equal to or less than $10^{23}$ (photons/s/mrad$^2$/mm$^2$/0.1% bw).

# FIG.1

# FIG.2

```
              ┌──────────────┐
              │    Start     │
              └──────┬───────┘
                     │
   ┌─────────────────┴─────────────────┐        S1
   │      Measure scattering data of    │  ◄───
   │   X-ray  or neutron of rubber material │
   └─────────────────┬─────────────────┘
                     │
   ┌─────────────────┴─────────────────┐        S2
   │ Determine and visualize three-dimensional │ ◄───
   │  structure of filler in the rubber material │
   │     with reverse Monte Carlo method  │
   │        by using the scattering data  │
   └─────────────────┬─────────────────┘
                     │
   ┌─────────────────┴─────────────────┐        S3
   │        Obtain a slice image from   │  ◄───
   │     the three-dimensional structure │
   └─────────────────┬─────────────────┘
                     │
   ┌─────────────────┴─────────────────┐        S4
   │  Set an initial rubber material model from │ ◄───
   │    the slice image of the rubber material │
   └─────────────────┬─────────────────┘
                     │
   ┌─────────────────┴─────────────────┐        S5
   │        Set a subdivision region    │  ◄───
   │    in the initial rubber material model │
   └─────────────────┬─────────────────┘
                     │
   ┌─────────────────┴─────────────────┐        S6
   │      Divide individual basic elements of │ ◄───
   │    the subdivision region into two or more │
   └─────────────────┬─────────────────┘
                     │
              ┌──────┴───────┐
              │     End      │
              └──────────────┘
```

FIG.3

## FIG.4(a)

## FIG.4(b)

## FIG.5(a)

## FIG.5(b)

# FIG.6

# FIG.7

## INTERNATIONAL SEARCH REPORT

| International application No. |
|---|
| PCT/JP2012/077888 |

**A. CLASSIFICATION OF SUBJECT MATTER**

*G01N23/201*(2006.01)i, *C08K3/00*(2006.01)i, *C08L21/00*(2006.01)i, *G01N3/00* (2006.01)i, *G01N23/202*(2006.01)i, *G01N33/44*(2006.01)i

According to International Patent Classification (IPC) or to both national classification and IPC

**B. FIELDS SEARCHED**

Minimum documentation searched (classification system followed by classification symbols)
G01N23/00-23/227, C08K3/00, C08L21/00, G01N3/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)
JSTPlus/JST7580(JDreamII)

**C. DOCUMENTS CONSIDERED TO BE RELEVANT**

| Category* | Citation of document, with indication, where appropriate, of the relevant passages | Relevant to claim No. |
|---|---|---|
| Y | JP 2006-138810 A  (Sumitomo Rubber Industries, Ltd.), 01 June 2006 (01.06.2006), entire text; all drawings & US 2006/0106586 A1    & EP 1657657 A2 & KR 10-2006-0054142 A   & CN 1776696 A & TWB 00I344611 | 1-3 |
| Y | Yoshiyuki AMEMIYA et al., "Choki Riyo Kadai Hokoku 2 Nijigen Kyokushokaku · Shokaku X-sen Sanranho o Mochiita Gum Chu Nano Ryushi Gyoshu Kozo no Kansatsu", SPring-8 Riyosha Joho, 16 May 2009 (16.05.2009), vol.14, no.2, 149 to 153 | 1-3 |

☒ Further documents are listed in the continuation of Box C.     ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 21 January, 2013 (21.01.13) | 05 February, 2013 (05.02.13) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

| International application No. |
| --- |
| PCT/JP2012/077888 |

C (Continuation). DOCUMENTS CONSIDERED TO BE RELEVANT

| Category* | Citation of document, with indication, where appropriate, of the relevant passages | Relevant to claim No. |
| --- | --- | --- |
| A | JP 10-011613 A (Matsushita Electric Works, Ltd.), 16 January 1998 (16.01.1998), abstract; paragraph [0012] (Family: none) | 1-3 |
| A | JP 2005-121535 A (Sumitomo Rubber Industries, Ltd.), 12 May 2005 (12.05.2005), abstract; paragraphs [0047], [0052] & US 2005/0086034 A1  & EP 1526468 A2 & DE 602004023360 D  & KR 10-2005-0037342 A & CN 1609884 A  & TWB 00I339263 | 1-3 |
| A | JP 2008-122154 A (Sumitomo Rubber Industries, Ltd.), 29 May 2008 (29.05.2008), entire text; all drawings (Family: none) | 1-3 |
| A | JP 2009-003747 A (The Yokohama Rubber Co., Ltd.), 08 January 2009 (08.01.2009), entire text; all drawings (Family: none) | 1-3 |
| A | Hiromichi KISHIMOTO, "Gum Fukugo Zairyo eno Hoshako no Oyo to Kaiseki", Sentan Sen'i Sozai Kenkyu Iinkai Koenkai Yoshishu (AFMc), 2008, vol.32, 35 to 40 | 1-3 |
| A | Yuya SHINOHARA et al., "Small-angle X-ray scattering of filled rubber", Function & materials, 05 March 2007 (05.03.2007), vol.27, no.4, 83 to 90 | 1-3 |

Form PCT/ISA/210 (continuation of second sheet) (July 2009)